# EUROPÄISCHE PATENTSCHRIFT

(11) **EP 0 931 528 B1**
(45) Veröffentlichungstag und Bekanntmachung des Hinweises auf die Patenterteilung: **23.07.2008**
(21) Anmeldenummer: 99100158.7
(22) Anmeldetag: 07.01.1999
(51) Int. Cl.: A61F 13/06

(54) **Bandage für das Sprunggelenk**
Ankle bandage
Bandage pour la cheville

(30) Priorität: 23.01.1998 DE 19802511
(43) Veröffentlichungstag der Anmeldung: 28.07.1999
(73) Patentinhaber: Beiersdorf Aktiengesellschaft, 20245 Hamburg (DE)
(72) Erfinder: Bodenschatz, Stefan Dr., 21614 Buxtehude (DE); Rosenbaum, Brigitte, 21079 Hamburg (DE)

(56) Entgegenhaltungen:
- DE-A- 3 122 463
- FR-A- 2 364 647
- US-A- 4 367 733
- US-A- 4 753 228

## Beschreibung

Die Erfindung betrifft eine Bandage für das Sprunggelenk.

Orthopädischen Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigen Material, zum Beispiel aus Neopren, Gewirke, oder Geweben.
Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.

Das Zusammenfügen kann durch Nähen, Kleben oder andere übliche Verfahren erfolgen. Der große Nachteil dieser Bandagen ist, daß die genaue, anatomische Paßform nur schwierig erreicht werden kann und eine Vielzahl von Verbindungsstellen entstehen, beispielsweise Nähte. Diese Verbindungsstellen verändern die Eigenschaften des eingesetzten Materials, und es besteht die Gefahr von Druckstellen auf der Haut.

Sprunggelenkorthesen oder -bandagen dienen bevorzugt für die frühfunktionelle Behandlung frischer fibularer Bandrupturen und von leichten und mittelschweren Fußwurzeldistorsionen, als auch zur Anwendung bei chronischer Instabilität.

Durch die DE-A-38 40 714 ist eine Sprunggelenkorthese mit einem U-förmigen Stützbügel bekannt, dessen Schenkel unterhalb des Fußes in einem Steg zusammenlaufen, über die Knöchel hinaufreichen und in ihrem Endbereich durch ein Befestigungsband zusammen gehalten sind. Dabei ist der äußere Schenkel seitlich vor seinem Knöchel und der innere Schenkel in Gegenüberstellung zum äußeren Schenkel vor der Achillessehne hochgeführt. Beide Schenkel sind in Richtung zu dem Steg bis zu einer Lage vor der Ferse geführt und verlaufen in Richtung zu ihren Enden aufwärts derart, daß sie seitlich neben den Schienbeinkanten etwa parallel zu diesen aufwärts streben, wobei im unteren Bereich der Schenkel ein Halteband angebracht ist, das von dem einen Schenkel über den Rist schräg aufwärts zum anderen Schenkel an diesen festlegbar verläuft, oberhalb der Knöchel um die Achillessehne greift und auf den Rist sich überkreuzend an dem anderen Schenkel in einem Halteteil endet. Mit einer derart ausgebildeten Sprunggelenkorthese soll das Umknicken vor allem in Richtung seitvorn, also in Richtung auf eine Spitzfuß-Stellung, verhindert werden. Da der U-förmige Stützbügel dieser Sprunggelenkorthese mit seinem äußeren Schenkel seitlich vor dem Fußknöchel und mit seinem inneren Schenkel vor der Achillessehne hochgeführt und von einem unterhalb des Fußes verlaufenden Steg zusammengehalten sind, wird der mediale Rand des Mittelfußes nicht erfaßt, so daß der Einsatz dieser Sprunggelenkorthese begrenzt ist.

Eine Fußfixierungsschiene beschreibt die DE-A-39 09 922. Diese Fußfixierungsschiene dient insbesondere zur postoperativen Behandlung eines verletzten Sprunggelenkes mit einem den Fuß umfassenden Fußteil, an den sich nach oben bis in den Wadenbereich reichend ein mit Verschlußzügeln versehener Halteteil anschließt. Der Halteteil ist dabei in zwei Seitenteile unterteilt, die an den Fußteil anschließen und schalenförmig ausgebildet sind. Jeweils der den Fußknöchel überdeckende Bereich des Seitenteils ist mit einer fensterartigen Ausnehmung versehen. Der Bereich der Achillessehne am Fußteil und am Halteteil ist dabei ausgespart. Die einstell- und feststellbaren bandförmigen Verschlußzügel bestehen aus einem undehnbaren Material, wobei ein Verschlußzügel an dem Fußteil so angeordnet ist, daß er den Fußrücken übergreifend den ersten Strahl des Mittelfußes gegen supinatorisches Auf steigen fixiert. Mit einer derartigen Fußfixierungsschiene soll zum einen eine einwandfreie Ruhigstellung des zu behandelnden Fußes erreicht werden, und zum anderen sollen die Nachteile eines Gipsverbandes vermieden werden, denn nach Verletzungen und Operationen am äußeren Bandapparat wird oftmals der Fuß zur Ruhigstellung eingegipst, wobei eine postoperative Behandlung von Operationswunden wegen zahlreicher gravierender Nachteile nicht möglich ist. Bei dieser Fußfixierungsschiene wird von einer U-förmigen Gelenkmanschette mit vollflächigem Sohlenteil ausgegangen, das den Mittel- und Vorfuß bis zum kleinen Zehenballen erfaßt, wobei jedoch keine ausreichende Gelenkigkeit im Mittelfußbereich gegeben ist.

Die DE-C-43 18 588 offenbart ebenfalls eine Sprunggelenkorthese, die aus einer U-förmigen, aus thermoplatischem Material hergestellten Gelenkmanschette besteht, die sich aus einer Außenknöchelschiene und einer Innenknöchelschiene zusammensetzt. Diese Knöchelschienen sind durch einen unter der Ferse verlaufenden Steg verbunden. Die Knöchelschienen weisen ferner anatomisch gerechte Vertiefungen zur Anpassung an die Knöchelkonturen und zum anatomiegerechten Sitz auf. Ein weiteres Element der Orthese ist sein Mittelfußteil, das ebenfalls aus thermoplastischem Material gefertigt ist. Dieser Teil der Orthese läuft quer unter der Fußsohle proximal der Köpfchen der Mittelfußknochen I-V und ist medial und lateral laschenförmig ausgebildet. Die so ausgebildeten Laschen umfassen die Fußränder außen und innen. Sie führen zum einen den Mittelfuß, zum anderen dienen sie zur Befestigung von Kreuz- und Quergurtbändern. Das Mittelfußteil ist dabei fußsohlenseitig durch einen ebenfalls aus thermoplastischem Material hergestellten, jedoch hochflexiblen Steg verbunden. Dieser Steg hat die Funktion eines Gelenkes und arbeitet analog einem Filmscharnier. Die Drehachse dieses in dem hochflexiblen Steg ausgebildeten Gelenkes verläuft von dorso-medial nach antero-lateral und bildet mit der Fußlängsachse einen Winkel von etwa 10°, und zwar entsprechend der Anatomie des unteren Sprunggelenks.

DE 3122463-A1 und US 4367733 offenbaren eine Sprunggelenkbandage, bestehend aus einem länglichen Streifen, wobei die erste Querkante des länglichen Streifens am länglichen Streifen selbst befestigt ist. Zudem weise die Bandagen beider Druckschriften einen Zügel auf, der an dem länglichen Streifen befestigt ist. Die Bandagen nach DE 3122463-A1 weisen für das Sprunggelenk als zusätzliche Ausstattungsmerkmale zwei beidseitig mit Klettverschlüssen versehene Haftstreifen und zwei Fixier- und Stützstreifen, um einen guten Sitz am Fuß sowie eine ausreichende Stabilisierung des Gelenks zu gewährleisten.
Die Bandage nach US 4367733 benötigt ein Befestigungsmaterial zur Sicherung, dass mit zusätzlichen Nähten befestigt ist. Weiterhin benötigt die Bandage zwingend diverse Befestigungszügel.

Der Erfindung liegt die Aufgabe zugrunde, eine Bandage zu schaffen, die insbesondere am Sprunggelenk einzusetzen und die als solche für eine funktionelle Behandlung von leichten und mittelschweren Fußwurzeldistorsionen und chronischen Instabilitäten geeignet ist, mit der eine laterale und mediale Stabilisierung vom oberen und unteren Sprunggelenk erreicht wird, wodurch das Risiko sowohl für Inversionstraumata als auch Eversionstraumata verringert wird.

Diese Aufgabe wird durch die gemäß Hauptanspruch gekennzeichnete Bandage gelöst. Gegenstand der Unteransprüche sind dabei vorteilhafte Weiterbildungen der Bandage.

Demgemäß besteht die Bandage für das Sprunggelenk aus einem länglichen Steifen und einem an dem länglichen Streifen befestigten Zügel. Die erste Querkante des Streifens ist im wesentlichen vertikal auf der medialen Seite des Sprunggelenks angeordnet. Ausgehend von der ersten Querkante wird der längliche Streifen um die Ferse, über die laterale Seite des Sprunggelenks, über den Fußrücken nach medial plantar und über die Fußsohle nach lateral plantar geführt. Die erste Querkante des länglichen Streifens ist am länglichen Streifen selbst befestigt, und zwar vernäht..

Des weiteren setzt an der zweiten Querkante der Zügel an, der ausgehend von der lateralen Seite der Fußsohle über den Fußrücken zur medialen Seite des Sprunggelenks, um die Ferse zur lateralen Seite des Sprunggelenks und über die laterale Seite des Sprunggelenks hinaus geführt und befestigt wird, wobei die zweite Querkante am länglichen Streifen vernäht ist.

Diese geschilderte Art der Anordnung des länglichen Streifens führt dazu, dass der längliche Streifen in Schraubenform mit annähernd zwei Gewindegängen vorgelegt und bevorzugt vernäht wird, so dass sich ein hohles Inneres innerhalb der Bandage zur Aufnahme des Fußes ergibt.

Der längliche Streifen weist in einer bevorzugten Ausführungsform eine Querelastizität auf von 0% bis 100%, insbesondere von 5% bis 30%, und eine Längselastizität von 30% bis 250%, insbesondere von 80% bis 120%.

Der Zügel weist in einer weiteren bevorzugten Ausführungsform eine Querelastizität auf von 0% bis 100%, insbesondere von 5% bis 30%, und eine Längselastizität von 30% bis 250%, insbesondere von 50% bis 100%.

Als besonders vorteilhaft hat es sich erwiesen, wenn der längliche Streifen etwa 40 bis 60 cm und der Zügel etwa 25 bis 40 cm lang und der längliche Streifen etwa 8 bis 12 cm und der Zügel etwa 6 bis 10 cm breit sind.

In einer weiteren bevorzugten Ausführungsform der Bandage ist die zweite Querkante zumindest mit dem proximalen Ende am länglichen Streifen befestigt ist, und es setzt an der zweiten Querkante der Zügel an.

Dann ist weiterhin eine Ausführungsform bevorzugt, in der die zweite Querkante vollständig am länglichen Streifen befestigt ist und an der zweiten Querkante darüber hinaus der Zügel ansetzt.

Der erfindungsgemäße Aufbau der Bandage ermöglicht, daß der längliche Streifen und der Zügel aus dem gleichen Material bestehen und insbesondere in einem einzigen Zuschnitt ausgestanzt werden.

Durch die erfindungsgemäßen Merkmale der Bandage bewirkt diese eine zirkuläre Kompression und eine laterale und mediale Stabilisierung von oberem und unterem Sprunggelenk, das heißt, es wird das Risiko sowohl für Inversionstraumata als auch für Eversionstraumata verringert.
Die Bandage erlaubt einen hohen Grad der Mobilisierung, wie es für die funktionelle Behandlung des Sprunggelenks erforderlich ist; sie garantiert aber auch durch die definierte, begrenzte Elastizität eine sichere Stabilisierung des verletzten Sprunggelenks. Die Bandage ist deshalb besonders geeignet für funktionelle Behandlung von leichten und mittelschweren Fußwurzeldistorsionen und chronischen Instabilitäten.

Die Erfindung wird anhand von vier schematischen Zeichnungen eines Ausführungsbeispiels näher erläutert, und zwar die Ausführung für den linken Fuß, ohne damit die Erfindung unnötig einschränken zu wollen. Die Ausführungsvariante der Bandage für den rechten Fuß ist zu der ersteren spiegelsymmetrisch ausgeführt. In der bevorzugten Ausführungsform kann durch Umstülpen der Bandage diese von der linken in die rechte Ausführungsvariante überführt werden.

Es zeigen
- Figur 1: den Vorgang des Anlegens der Bandage zum Beginn, wenn der Fuß in den Streifen schlüpfen soll,
- Figur 2 bis Figur 4: den Vorgang des Anlegens des Zügels, der am Streifen befestigt ist.

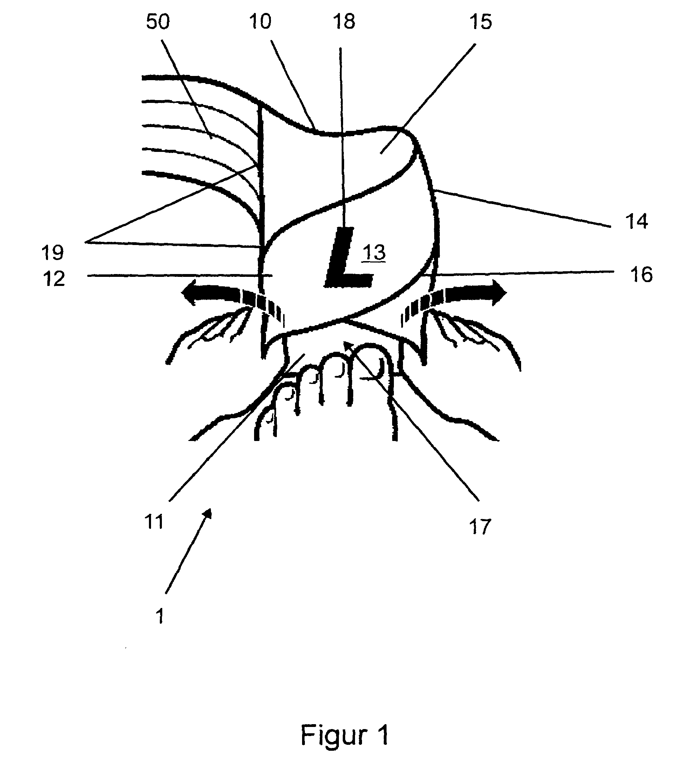

In der Figur 1 ist eine besonders vorteilhafte Ausführungsform der erfindungsgemäßen Bandage 1 für das Sprunggelenk dargestellt. Die Bandage 1 setzt sich demgemäß aus einem länglichen Steifen 10 und einem an dem länglichen Streifen 10 befestigten Zügel 50 zusammen.

Die erste Querkante 16 des Streifens 10 ist im wesentlichen vertikal auf der medialen Seite des Sprunggelenks angeordnet. Ausgehend von der ersten Querkante 16 wird der längliche Streifen 10 um die Ferse (Abschnitt 11), über die laterale Seite des Sprunggelenks (Abschnitt 12), über den Fußrücken nach medial plantar (Abschnitte 13 und 14) und über die Fußsohle nach lateral plantar (Abschnitt 15) geführt.
Die erste Querkante 16 des länglichen Streifens 10 ist am länglichen Streifen 10 selbst vernäht.

Die zweite Querkante 19 ist zum einen mit dem proximalen Ende am länglichen Streifen 10 vernäht. Zum anderen setzt an der zweiten Querkante 19 ebenfalls der Zügel 50 an.

Auf dem Teil der Bandage 1, der über dem Fußrücken liegt (Abschnitt 13), ist vorteilhafterweise mittels eines Etiketts oder eines Aufdrucks 18 angegeben, an welchem Sprunggelenk die Bandage 1 anzulegen ist, und zwar in diesem Falle "L" für den linken Fuß.

Durch die spezielle Art der Führung der Bandage 1 ergibt sich die Öffnung 17, in dem nach dem Anlegen der Bandage 1 der Fesselbereich des Patientenbeines steckt.

Die Figur 1 zeigt, wie der Fuß des Patienten in die Bandage 1 eingeführt wird. Die Bandage 1 wird dazu - wie dargestellt - an der Öffnung 17 gedehnt., so daß der Fuß in den strumpfähnlichen Bereich der Bandage 1 schlüpfen kann.

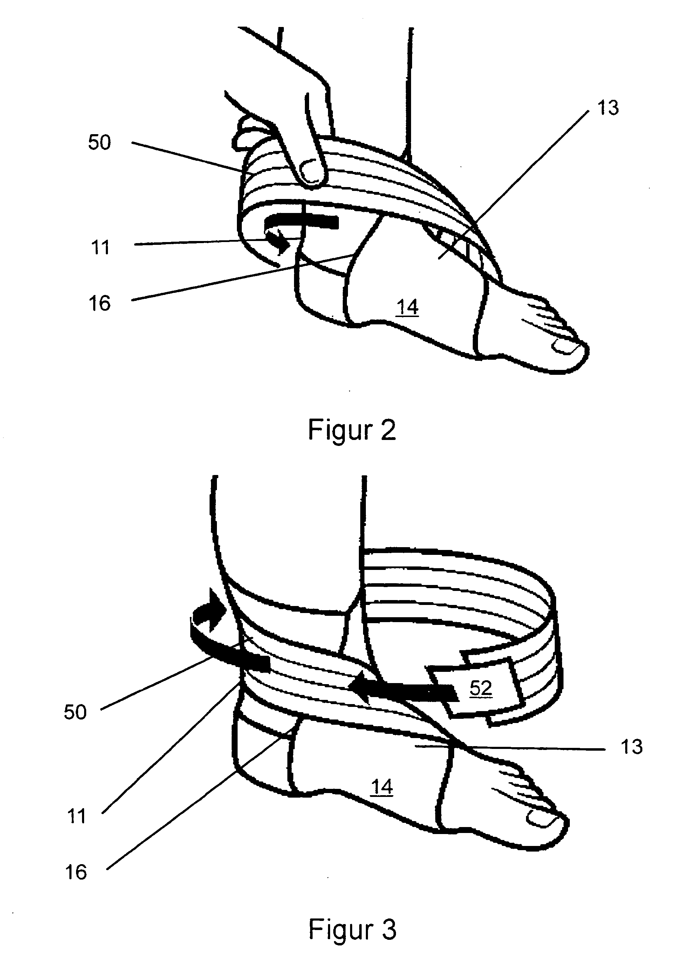

In den Figuren 2 bis 4 ist das Anlegen des Zügels 50 gezeigt, nachdem der Fuß in den strumpfähnlichen Bereich der Bandage 1, der vom Streifen 10 gebildet wird, eingeführt worden ist.

Der Zügel 50 wird - gemäß Figur 2 - ausgehend von der lateralen Seite der Fußsohle über den Fußrücken zur medialen Seite des Sprunggelenks geführt. Figur 3 stellt dar, daß der Zügel um die Ferse zur lateralen Seite des Sprunggelenks und über die laterale Seite des Sprunggelenks hinaus geführt wird.

An dem Zügel 50 ist bevorzugt ein Etikett 52 vorgesehen, das nach Art eines Klettverschlusses beim Umwickeln des Beins mit dem Zügel 50 die Befestigung des Zügels auf sich selbst sicherstellt.

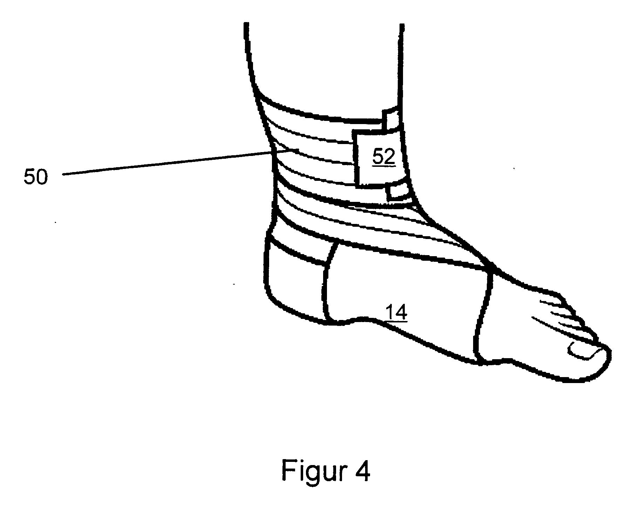

In der Figur 4 ist schließlich die perfekt das Sprunggelenk umgebende Bandage 1 mit gesichertem Ende des Zügels 50 gezeigt, nachdem der Zügel 50 ein weiteres Mal um das Bein des Patienten gewickelt worden ist.

## Patentansprüche

1. Bandage 1 für das Sprunggelenk, bestehend aus einem länglichen Streifen 10 und einem an dem länglichen Streifen 10 befestigten Zügel 50, wobei die erste Querkante 16 des länglichen Streifens 10 im wesentlichen vertikal auf der medialen Seite des Sprunggelenks angeordnet ist, der längliche Streifen 10 um die Ferse (Abschnitt 11), über die laterale Seite des Sprunggelenks (Abschnitt 12), über den Fußrücken nach medial plantar (Abschnitte 13 und 14) und über die Fußsohle nach lateral plantar (Abschnitt 15) geführt wird, wobei die erste Querkante 16 des länglichen Streifens 10 am länglichen Streifen 10 selbst befestigt ist und wobei an der zweiten Querkante 19 der Zügel 50 ansetzt, der ausgehend von der lateralen Seite der Fußsohle über den Fußrücken zur medialen Seite des Sprunggelenks, um die Ferse zur lateralen Seite des Sprunggelenks und über die laterale Seite des Sprunggelenks hinaus geführt und befestigt wird, **dadurch gekennzeichnet, dass** die zweite Querkante 19 am länglichen Streifen 10 vernäht ist.

2. Bandage nach Anspruch 1, **dadurch gekennzeichnet, dass** der längliche Streifen 10 eine Querelastizität aufweist von 0% bis 100%, insbesondere von 5% bis 30%, und eine Längselastizität von 30% bis 250%, insbesondere von 80% bis 120%.

3. Bandage nach Anspruch 1, **dadurch gekennzeichnet, dass** der Zügel 50 eine Querelastizität aufweist von 0% bis 100%, insbesondere von 5% bis 30%, und eine Längselastizität von 30% bis 250%, insbesondere von 50% bis 100%.

4. Bandage nach Anspruch 1, **dadurch gekennzeichnet, dass** der längliche Streifen 10 etwa 40 bis 60 cm und der Zügel 50 etwa 25 bis 40 cm lang sind, der längliche Streifen 10 etwa 8 bis 12 cm und der Zügel 50 etwa 6 bis 10 cm breit sind.

5. Bandage nach Anspruch 1, **dadurch gekennzeichnet, dass** die zweite Querkante 19 zumindest mit dem proximalen Ende am länglichen Streifen 10 befestigt ist und an der zweiten Querkante 19 der Zügel 50 ansetzt.

6. Bandage nach Anspruch 1, **dadurch gekennzeichnet, dass** die zweite Querkante 19 vollständig am länglichen Streifen 10 befestigt ist und an der zweiten Querkante 19 darüber hinaus der Zügel 50 ansetzt.

7. Bandage nach Anspruch 1, **dadurch gekennzeichnet, dass** der längliche Streifen 10 und der Zügel 50 aus dem gleichen Material bestehen und insbesondere in einem einzigen Zuschnitt ausgestanzt werden.

8. Bandage nach Anspruch 1, **dadurch gekennzeichnet, dass** durch Umstülpen der Bandage diese von der linken in die rechte Ausführungsvariante überführt werden kann.

## Claims

1. Bandage 1 for the ankle joint, consisting of an elongate strip 10 and of a strap 50 secured on the elongate strip 10, the first transverse edge 16 of the elongate strip 10 being arranged essentially vertically on the medial side of the ankle joint, the elongate strip 10 being guided round the heel (section 11), across the lateral side of the ankle joint (section 12), across the back of the foot in the medial-plantar direction (sections 13 and 14) and across the sole of the foot in the lateral-plantar direction (section 15), the first transverse edge 16 of the elongate strip 10 being secured on the elongate strip 10 itself, and the strap 50 being joined to the second transverse edgel9, the said strap 50 being guided from the lateral side of the sole of the foot across the back of the foot to the medial side of the ankle joint, round the heel to the lateral side of the ankle joint and across the lateral side of the ankle joint and being secured, **characterized in that** the second transverse edge 19 is stitched on the elongate strip 10.

2. Bandage according to Claim 1, **characterized in that** the elongate strip 10 has a transverse elasticity of 0% to 100%, in particular 5% to 30%, and a longitudinal elasticity of 30% to 250%, in particular 80% to 120%.

3. Bandage according to Claim 1, **characterized in that** the strap 50 has a transverse elasticity of 0% to 100%, in particular 5% to 30%, and a longitudinal elasticity of 30% to 250%, in particular 50% to 100%.

4. Bandage according to Claim 1, **characterized in that** the elongate strip 10 is approximately 40 to 60 cm long and the strap 50 is approximately 25 to 40 cm long, and the elongate strip 10 is approximately 8 to 12 cm wide and the strap 50 is approximately 6 to 10 cm wide.

5. Bandage according to Claim 1, **characterized in that** the second transverse edge 19 is secured on the elongate strip 10 at least with the proximal end and the strap 50 is joined to the second transverse edge 19.

6. Bandage according to Claim 1, **characterized in that** the second transverse edge 19 is secured completely on the elongate strip 10 and the strap 50 is also joined to the second transverse edge 19.

7. Bandage according to Claim 1, **characterized in that** the elongate strip 10 and the strap 50 are made of the same material and in particular are punched out in a single blank.

8. Bandage according to Claim 1, **characterized in that** by turning the bandage inside out, the bandage can be converted from the left-foot version to the right-foot version.

## Revendications

1. Bandage 1 pour la cheville, constitué d'une bande allongée 10 et d'une bride 50 fixée à la bande allongée 10, la première arête transversale 16 de la bande allongée 10 étant disposée essentiellement verticalement sur le côté interne de la cheville, la bande allongée 10 étant passée autour du talon (portion 11), par-dessus le côté latéral de la cheville (portion 12), par-dessus le coup de pied vers la direction plantaire interne (portions 13 et 14) et par-dessus la plante du pied vers la direction plantaire latérale (portion 15), la première arête transversale 16 de la bande allongée 10 étant fixée à la bande allongée 10 elle-même et la bride 50 se rattachant à la deuxième arête transversale 19 et étant guidée et fixée, en partant du côté latéral de la plante du pied en passant par-dessus le coup de pied jusqu'au côté interne de la cheville, autour du talon vers le côté latéral de la cheville et par-dessus le côté latéral de la cheville, **caractérisé en ce que** la deuxième arête transversale 19 est cousue à la bande allongée 10.

2. Bandage selon la revendication 1, **caractérisé en ce que** la bande allongée 10 présente une élasticité transversale de 0 % à 100 %, notamment de 5 % à 30 %, et une élasticité longitudinale de 30 % à 250 %, notamment de 80 % à 120%.

3. Bandage selon la revendication 1, **caractérisé en ce que** la bride 50 présente une élasticité transversale de 0 % à 100 %, notamment de 5 % à 30 %, et une élasticité longitudinale de 30 % à 250 %, notamment de 50 % 100 %.

4. Bandage selon la revendication 1, **caractérisé en ce que** la bande allongée 10 mesure environ 40 à 60 cm de long et la bride 50 environ 25 à 40 cm de long, et la bande allongée 10 mesure environ 8 à 12 cm de large et la bride 50 environ 6 à 10 cm de large.

5. Bandage selon la revendication 1, **caractérisé en ce que** la deuxième arête transversale 19 est fixée au moins par l'extrémité proximale à la bande allongée 10 et se rattache à la deuxième arête transversale 19 de la bride 50.

6. Bandage selon la revendication 1, **caractérisé en ce que** la deuxième arête transversale 19 est complètement fixée à la bande allongée 10 et en outre la bride 50 se rattache à la deuxième arête transversale 19.

7. Bandage selon la revendication 1, **caractérisé en ce que** la bande allongée 10 et la bride 50 se composent du même matériau et en particulier sont découpées dans une pièce unique.

8. Bandage selon la revendication 1, **caractérisé en ce que** l'on peut passer d'une forme de réalisation gauche à une forme de réalisation droite en retournant le bandage.
